Europäisches Patentamt

(19) European Patent Office

Office européen des brevets

(11) Publication number: **0 083 360 B1**

(12) **EUROPEAN PATENT SPECIFICATION**

(45) Date of publication of patent specification: **15.04.87**

(51) Int. Cl.⁴: **A 61 K 31/70,** A 61 K 33/06, A 61 K 33/14

(21) Application number: **82902287.0**

(22) Date of filing: **07.06.82**

(86) International application number: **PCT/US82/00774**

(87) International publication number: **WO 83/00087 20.01.83 Gazette 83/02**

(54) PERITONEAL DIALYSES SOLUTION CONTAINING CARBOHYDRATE POLYMERS.

(30) Priority: **10.07.81 US 282309**

(43) Date of publication of application: **13.07.83 Bulletin 83/28**

(45) Publication of the grant of the patent: **15.04.87 Bulletin 87/16**

(84) Designated Contracting States: **BE CH DE FR GB LI SE**

(56) References cited:
US-A-3 525 686
US-A-3 911 915
US-A-3 928 135
US-A-4 182 756
US-A-4 308 255

Trans, AM. Soc. Int. Organs, Vol. 18, Pages 423-428, issued 17 April 1972 Ahearn, D. J. et al. "Addition Of aminoacids/to Peritoneal-Dialysis fluid" Lancet, p. 812, issued 12 October 1968 ACTA MED SCAND vol. 185 (1969) p. 237-239

(73) Proprietor: **BAXTER TRAVENOL LABORATORIES, INC.**
**One Baxter Parkway**
**Deerfield, IL 60015 (US)**

(72) Inventor: **ALEXANDER, Steven R.**
**9837 SW Kimberley Drive**
**Tigard, OR 97223 (US)**
Inventor: **MYERS, W. Michael**
**17810 SW Shasta Trail**
**Tualatin, OR 97062 (US)**

(74) Representative: **Popp, Eugen, Dr. et al**
**MEISSNER, BOLTE & PARTNER**
**Widenmayerstrasse 48 Postfach 86 06 24**
**D-8000 München 86 (DE)**

Courier Press, Leamington Spa, England.

## Description

The medical procedure known as continuous ambulatory peritoneal dialysis (CAPD) is rapidly growing in clinical acceptance as the technique of choice for maintaining many patients who have lost kidney function. Peritoneal dialysis solution is inserted into the peritoneal cavity, whereby diffusion exchange takes place between the solution and the bloodstream across the natural body membranes, to remove by diffusion the waste products which are normally excreted through the kidneys, typically solutes such as sodium and chloride ions and the other materials normally excreted by the body such as urea, creatinine, and water.

The nature and rate of the materials removed from the body by peritoneal dialysis is a function of the solutes present in the peritoneal dialysis solution. Physiological salts are present in the peritoneal dialysis solution such as sodium chloride, calcium chloride, sodium lactate, and sodium acetate, generally at slightly hypotonic concentrations, except for the calcium, so that excess concentrations of such salts in the bloodstream will diffuse into the peritoneal dialysis solution for removal.

To remove water from the patient, as is generally necessary, other solutes may be added to generate the necessary osmotic pressure. Typically, this solute is a sugar such as glucose, which may normally be present in peritoneal dialysis solutions in a concentration of at least 0.5 percent by weight. When it is desired to increase the ultrafiltration of water from the patient, higher concentrations of sugar are used.

However, as a disadvantage of this system, during the peritoneal dialysis process, as water diffuses into the peritoneal dialysis solution, sugar present in the peritoneal dialysis solution diffuses into the bloodstream to a significant extent. Accordingly, while the system is safe and effective for increasing the ultrafiltration during peritoneal dialysis, it has certain disadvantages. For example, since the sugar diffuses relatively rapidly from the solution in the peritoneal cavity into the bloodstream, there is a considerable and rapid decrease in the osmolarity of the peritoneal dialysis solution. Accordingly, to obtain the desired amount of ultrafiltration, the initial concentration of sugar in the peritoneal dialysis solution must be relatively high to account for the fact that the osmolarity will fall by diffusion of sugar into the bloodstream.

Particularly in certain pediatric cases, children who are on a CAPD regime often lose significant appetite and fail to adequately gain weight. Accordingly it becomes desirable to administer substantial amounts of calories to the child. With the peritoneal dialysis solutions of this invention, desired calories can be administered to the pediatric patient while at the same time excessive concentrations of sugar per se are avoided. Such excessive concentrations of sugar could, of course, unduly raise the osmolarity of the solution and would provide undesirable levels of ultrafiltration to the patient. With the peritoneal dialysis solutions of this invention, the administration of substantial amounts of calories to the patient can be effected by diffusion from the peritoneal dialysis solution, simultaneously with the maintenance of a desired ultrafiltration rate, and also clearance of metabolic waste products such as urea and creatinine through the peritoneal cavity into the dialysis solution. By a proper balance between sugar and other ingredients as described in this invention in a peritoneal dialysis solution, a proper balance of calories, coupled with a proper rate of ultrafiltration can be provided.

In Ramsay et al. U.S. Patent No. 4,182,756, it is suggested to use high calorie solutions of low molecular weight glucose polymer mixtures in intravenous administration, since such solutions can provide significant increases in calories per liter over monomeric sugar solutions without being hypertonic. Other related prior art is cited in the same patent.

Milner U.S. Patent No. 3,928,135 also discusses glucose polymers as ingredients for oral ingestion or intravenous administration.

Seifter et al. U.S. Patent No. 3,911,915 teaches the dialytic introduction of maltose (a disaccharide) intraperitoneally into warm blooded animals. However, maltose shares in the disadvantages of glucose in that the addition of substantial amounts thereof can result in significant and excessive osmolarity so that inadequate amounts of calories may be provided to the pediatric patient by the peritoneal dialysis route, if the osmolarity is proper.

Raj et al. U.S. Patent No. 4,308,255 discloses dialysis solutions containing dextran. Dextran is also susceptible of being metabolized in the blood stream only to a very low extent. In normal patients, the bulk of intravenously administered dextran (when used as a volume expander) is excreted via the kidneys. In view of this, dextran cannot be used as a dialyzate in a long term treatment of patients suffering from loss of kidney function, because this would cause an accumulation of dextran in the uremic patients.

In accordance with this invention, a peritoneal dialysis solution is provided which comprises a water solution of physiologically tolerable pH, having physiological salts and metabolizable starch hydrolysate type glucose polymers having an average degree of polymerization of at least 4, in concentrations sufficient to safely effect the removal of solutes and water from a patient by peritoneal dialysis.

Basically, the peritoneal dialysis solution of this invention is similar to conventional peritoneal dialysis solutions, which also are of physiologically tolerable pH and have physiological salts such as sodium chloride, calcium chloride and sodium acetate in appropriate concentrations. However, a novel feature of this invention is that the sugar of conventional peritoneal dialysis solution is either partially or completely replaced by metabolizable starch hydrolysate type glucose polymers which have an average degree of polymerization of at least 4; i.e., they typically constitute at least tetrasaccharides, while conventional sugars are either monomers such as glucose or fructose, or dimers such as sucrose or maltose.

As the result of this the metabolizable starch hydrolysate type glucose polymers exert their osmotic effect to enhance the ultrafiltration of water into the peritoneal dialysis solution, but at the same time they are of higher molecular weight than the sugars conventionally used in prior art peritoneal dialysis solutions, so that their rate of diffusion from the peritoneal dialysis solution through a body membrane into the bloodstream is significantly slower during the peritoneal dialysis procedure. However, that amount of starch hydrolysate type glucose polymer which does transfer to the bloodstream is metabolizable, so that it can be broken down by the body without ill effect.

Accordingly, since the diffusion of the starch hydrolysate type glucose polymer in the dialysis solution into the peritoneal cavity is relatively slower than the diffusion of mono and disaccharides, the decrease in the osmolarity of the dialysis solution during the course of the peritoneal dialysis procedure is slower in the presence of such glucose polymers than in the peritoneal dialysis solutions of the prior art. This, in turn, means that lower initial osmolarities may be utilized in the peritoneal dialysis solutions of this invention, compared with those of the prior art, while still achieving equal ultrafiltration rates over a predetermined period of time in peritoneal dialysis procedure such as CAPD.

Also, as stated above, large amounts of calories may be provided to the patient by the use of the glucose polymers in accordance with this invention, while at the same time the osmolarity and the ultrafiltration can be controlled in a manner which is relatively independent of the amount of potential calories administered to the patient, so that a peritoneal dialysis solution can be provided which is tailormade to provide optimum calories to the patient while also exhibiting optimum ultrafiltration characteristics. The independent control of both of the above parameters can be accomplished by appropriate adjustment of the concentration of glucose in the solution, coupled with a concentration of glucose polymers in accordance with this invention, with additional control being provided by appropriate selection of the degree of polymerization of the glucose polymers of this invention. In other words, in some circumstances starch hydrolysate type glucose polymers which are tetrasaccharides or pentasaccharides may be used. In other cases, octasaccharides may be used.

Preferably, glucose polymers may be used in accordance with this invention. Such glucose polymers are commercially available, and are described in the patents discussed above. Mixtures of glucose polymers may be prepared by the hydrolysis of starch, and a substantial body of known prior art exists relating to the preparation and processing of glucose polymers.

For example, a glucose polymer may be used having an average degree of polymerization (number of saccharide units per molecule) of 5, in which at least 99 percent of its molecules have less than 26 glucose units; at least 85 percent of its molecules have less than 11 glucose units; and at least 20 percent of its molecules have less than 4 glucose units. However, if desired, starch hydrolysate type glucose polymers having different ranges of degree of polymerization may also be used. Substantially monodisperse polymers, having a relatively uniform degree of polymerization, may also be used if desired.

Typically, the peritoneal dialysis solution of this invention may comprise a water solution at a pH of 5 to 7.4 containing from 116 to 140 mEq/liter of sodium, 0 to 6 mEq/liter of calcium, 100 to 144 mEq/liter of chloride, and from 5 to 200 grams per liter of a metabolizable glucose polymer, preferably having an average degree of polymerization of 4 to 10. It is also desirable for from 30 to 45 mEq/liter of lactate or acetate to be present.

Also, other physiological ions such as magnesium, potassium, and carbonate may be present as desired, along with other additives which may have desirable benefit. For example, 0.5 to 25 grams per liter of amino acid salts or protein hydrolyzates may be added to further enhance the ultrafiltration of water into the peritoneal dialysis solution by their natural osmotic effect, and simultaneously to serve as a source of supplemental nitrogen for protein for the patient as they diffuse into the bloodstream. This can counterbalance the protein which the patient loses as a consequence of the peritoneal dialysis procedure, or may constitute the prime source of protein nutrition for the patient. Added sugars such as glucose, maltose, or dextrose may be present as well for purposes of nutrition, as well as creating an osmotic effect for enhancing ultrafiltration, for example 0.5 to 25 grams per liter.

The use of amino acids in peritoneal dialysis solutions is taught in the preliminary communication on page 812 of the October 12, 1968 issue of the *Lancet*. However, the peritoneal dialysis solutions disclosed there have no teaching of the use of metabolizable starch hydrolysate type glucose polymers in such solutions.

It is generally preferable for the pH of the solutions to be slightly on the acid side (5.4 to 6.8) to avoid caramelization of the starch hydrolysate type glucose polymers present during sterilization of the solution. However, the pH can be more alkaline than that with less ill effect during sterilization because of the polymeric nature of the sugar added thereto, which tends to stabilize it during the sterilization cycle.

Peritoneal dialysis solution concentrates may be made for later mixing with water to form the desired peritoneal dialysis solution of any desired concentration. Such concentrates may contain, for example, from 130 to 140 mEq/liter of sodium; from 3 to 4 mEq/liter of calcium; from 100 to 144 mEq/liter of chloride; and from 5 to 500 grams/liter of a metabolizable glucose polymer as described above. It is also desirable for from 30 to 40 mEq/liter of bicarbonate precursors such as one or more of lactate, acetate, malate, and/or succinate ions to be present. The bicarbonate precursor acid ions mentioned above, as well as other acid ions of the Krebs cycle may be added to also offer advantages in pH control of the peritoneal dialysis solution of this invention. The sodium or potassium salts of such ions, for example, may be used for this purpose, or the free acids. The above concentrate is preferably mixed with a conventional peritoneal dialysis solution. If mixed with water, higher ion concentrations would be desirable.

It is generally preferable for the osmolarity of the solutions of this invention to be from 272 to 700 milliosmols per liter, preferably 279 to 480 milliosmols per liter.

If amino acids or polypeptides are present in the solution, sulfhydryl-type antioxidants, for example N-acyl cysteine, may also be added to stabilize the amino acids in the peritoneal dialysis solution of this invention.

A typical solution which is contemplated for use in peritoneal dialysis is a sterile water solution containing the following: dextrose $H_2O$ — 15 grams per liter; sodium — 132 mEq/liter; calcium — 3.4 mEq/liter; chloride — 104 mEq/liter; lactate — 37 mEq/liter; glucose polymer having a degree of polymerization of greater than 4 (Polycose®, sold by Ross) — 120 grams per liter. This solution, when sterile, may be utilized as the peritoneal dialysis solution in a conventional CAPD procedure, utilizing the techniques and equipment developed and sold by the Artificial Organs Division of Baxter Travenol Laboratories, Inc., Deerfield, Illinois, so that good ultrafiltration may take place during the peritoneal dialysis procedure, with reduced diffusion of the glucose polymer into the bloodstream of the patient. Such a solution has an ultrafiltration capability equal to or greater than a commercially available peritoneal dialysis solution containing 4.25 weight percent of dextrose.

**Claims**

1. A peritoneal dialysis solution which comprises a water solution of physiologically tolerable pH, having physiological salts and metabolizable starch hydrolyzate type glucose polymers having an average degree of polymerization of at least 4 in concentrations sufficient to safely effect the removal of solutes and water from a patient by peritoneal dialysis.

2. A peritoneal dialysis solution which comprises a water solution of physiologically tolerable pH and containing 116 to 140 mEq/liter of sodium, 0 to 6 mEq/liter of calcium, 100 to 144 mEq/liter of chloride, and from 5 to 200 grams per liter of a metabolizable starch hydrolyzate type glucose polymer having an average degree of polymerization of 4 to 10.

3. The solution of Claim 2 in which from 30 to 45 mEq/liter of an ion selected from lactate, malate, acetate and succinate is present.

4. The solution of Claim 3 which has a pH of 5 to 7.4.

5. The solution of Claim 4 which comprises essentially 132 mEq/liter of sodium, 3.4 mEq/liter of calcium, 104 mEq/liter of chloride, 37 mEq/liter of lactate, and 120 grams/liter of said glucose polymer.

6. The solution of Claim 1 in which essentially 0.5 to 25 grams/liter of dextrose hydrate is present.

7. The solution of Claim 1 in which from 0.5 to 25 grams/liter of an amino acid source is present.

8. A peritoneal dialysis solution which comprises a water solution of pH 5 to 7.4 and containing 116 to 140 mEq/liter of sodium, 0 to 6 mEq/liter of calcium, 100 to 144 mEq/liter of chloride and from 5 to 200 grams per liter of metabolizable starch hydrolyzate type glucose polymer having an average degree of polymerization of at least 4, plus 30 to 45 mEq/liter of an ion selected from lactate, malate, acetate and succinate, and from 0.5 to 25 grams per liter of dextrose hydrate.

9. The solution of Claim 8 in which from 0.5 to 25 grams per liter of an amino acid source is present.

**Revendications**

1. Solution de dialyse péritonéale qui comprend une solution aqueuse ayant un pH physiologiquement tolérable, comportant des sels physiologiques et des polymères métabolisables de glucose du type hydrolysat d'amidon ayant un degré moyen de polymérisation d'au moins 4 en concentrations suffisantes pour effectuer sûrement l'extraction de solutés et d'eau d'un patient par dialyse péritonéale.

2. Solution de dialyse péritonéale qui comprend une solution aqueuse ayant un pH physiologiquement tolérable et contenant de 116 à 140 mEq/litre de sodium, de 0 à 6 mEq/litre de calcium, 100 à 144 mEq/litre de chlorure, et de 5 à 200 g/litre d'un polymère de glucose du type hydrolysat d'amidon métabolisable ayant un degré moyen de polymérisation compris entre 4 et 10.

3. Solution suivant la revendication 2, qui comprend de 30 à 45 mEq/litre d'un ion choisi parmi les lactate, malate, acétate et succinate.

4. Solution suivant la revendication 3, qui présente un pH compris entre 5 et 7,4.

5. Solution suivant la revendication 4, qui comprend sensiblement 132 mEq/litre de sodium, 3,4 mEq/litre de calcium, 104 mEq/litre de chlorure, 37 mEq/litre de lactate, et 120 g/litre du polymère de glucose précité.

6. Solution suivant la revendication 1, qui contient sensiblement de 0,5 à 25 g/litre d'hydrate de dextrose.

7. Solution suivant la revendication 1 qui contient de 0,5 à 25 g/litre d'une source aminoacide.

8. Solution péritonéale de dialyse qui com-

## 0 083 360

prend une solution aqueuse ayant un pH de 5 à 7,4 et contenant de 116 à 140 mEq/litre de sodium, de 0 à 6 mEq/litre de calcium, de 100 à 144 mEq/litre de chlorure et de 5 à 200 g/litre d'un polymère de glucose du type hydrolysat d'amidon métabolisable ayant un degré moyen de polymérisation d'au moins 4, plus 30 à 45 mEq/litre d'un ion choisi parmi les lactate, malate, acétate et succinate, et de 0,5 à 25 g/litre d'hydrate de dextrose.

9. Solution suivant la revendication 8, qui contient de 0,5 à 25 g/litre d'une source aminoacide.

**Patentansprüche**

1. Peritonealdialysierlösung, umfassend eine wäßrige Lösung mit physiologisch akzeptablem pH-Wert mit physiologischen Salzen und metabolisierbaren Stärkehydrolysat-Glucosepolymeren mit einem durchschnittlichen Polymerisationsgrad von wenigstens 4 in Konzentrationen, die ausreichen, um gelöste Stoffe und Wasser durch Peritonealdialyse sicher aus einem Patienten zu entfernen.

2. Peritonealdialysierlösung, umfassend eine wäßrige Lösung mit physiologisch akzeptablem pH-Wert, die 116 bis 140 mEq/l Natrium, 0 bis 6 mEq/l Calcium, 100 bis 144 mEq/l Chlorid und zwischen 5 und 200 g/l eines metabolisierbaren Stärkehydrolysat-Glucosepolymeren mit einem durchschnittlichen Polymerisationsgrad von 4 bis 10 enthält.

3. Lösung nach Anspruch 2, in der zwischen 30 und 45 mEq/l eines Ions vorhanden sind, das Laktat, Malat, Acetat oder Sukzinat ist.

4. Lösung nach Anspruch 3 mit einem pH-Wert von 5 bis 7,4.

5. Lösung nach Anspruch 4, im wesentlichen umfassend 132 mEq/l Natrium, 3,4 mEq/l Calcium, 104 mEq/l Chlorid, 37 mEq/l Laktat und 120 g/l des Glucosepolymeren.

6. Lösung nach Anspruch 1, in der im wesentlichen 0.5 bis 25 g/l Glukosehydrat vorhanden sind.

7. Lösung nach Anspruch 1, in der zwischen 0.5 und 25 g/l eines Aminosäure-Ausgangsstoffs vorhanden sind.

8. Peritonealdialysierlösung, umfassend eine wäßrige Lösung mit einem pH-Wert von 5 bis 7,4, die 116 bis 140 mEq/l Natrium, 0 bis 6 mEq/l Calcium, 100 bis 144 mEq/l Chlorid und zwischen 5 und 200 g/l eines metabolisierbaren Stärkehydrolysat-Glucosepolymeren mit einem durchschnittlichen Polymerisationsgrad von wenigstens 4 plus 30 bis 45 mEq/l eines Ions, das Laktat, Malat, Acetat oder Sukzinat ist, und zwischen 0.5 und 25 g/l Glucosehydrat enthält.

9. Lösung nach Anspruch 8, in der zwischen 0.5 und 25 g/l eines Aminosäure-Ausgangsstoffs vorhanden sind.